# FASCICULE DE BREVET EUROPEEN

(11) **EP 1 802 347 B1**
(45) Date de publication et mention de la délivrance du brevet: **10.06.2009**
(21) Numéro de dépôt: 05807395.8
(22) Date de dépôt: 05.10.2005
(51) Int. Cl.: A61K 47/24, A61K 47/36, A61K 9/00, A61P 11/00

(54) **COMPOSITION POUR LUTTER CONTRE LE RONFLEMENT**
ANTISCHNARCH-ZUSAMMENSETZUNG
ANTI-SNORING COMPOSITION

(30) Priorité: 20.10.2004 FR 0411153
(43) Date de publication de la demande: 04.07.2007
(73) Titulaire: PERSEE MEDICA, 78870 Bailly (FR)
(72) Inventeur: ANTON, Jean-Christophe, F-67000 Strasbourg (FR)
(74) Mandataire: Novagraaf IP
(86) Numéro de dépôt international: PCT/FR2005/002444
(87) Numéro de publication internationale: WO 2006/042926

(56) Documents cités:
- EP-A- 0 507 035
- WO-A-03/070178
- FR-A- 2 466 273
- KR-A- 2003 063 671
- US-A- 4 459 285
- PATENT ABSTRACTS OF JAPAN vol. 012, no. 446 (C-546), 24 novembre 1988 (1988-11-24) & JP 63 169931 A (NIPPON OIL & FATS CO LTD), 13 juillet 1988 (1988-07-13)
- LEONAVICIENE L ET AL: "AN IMPROVED MODEL OF OSTEOARTHRITIS IN RATS AND ITS THERAPY WITH THE TINCTURE OF FILIPENDULA ULMARIA (L.) MAXIM" BALTIC JOURNAL OF LABORATORY ANIMAL SCIENCE, COMPANY GRINDEX, RIGA, LV, vol. 11, no. 4, 2001, pages 225-237, XP001182640 ISSN: 1407-0944

## Description

La présente invention se rapporte au domaine du traitement du ronflement, et plus particulièrement à une composition permettant de lutter contre le ronflement.

Le ronflement est un bruit dû au passage de l'air dans des voies aériennes rétrécies au niveau du pharynx. Il traduit la vibration du voile du palais et des parois du pharynx sous l'action du flux aérien anormalement turbulent.

En effet, l'air inspiratoire traverse, entre la cavité nasale et la trachée, toutes deux rigides et indéformables, un espace déformable, élastique et plus ou moins rétréci. Dans ce rétrécissement vont apparaître des modifications de débit et de pression qui vont conduire à une vibration du voile du palais lié au claquement de celui-ci contre les parois du pharynx (phénomènes de Venturi). Pour que la vibration se produise, il faut que successivement, et environ 30 fois par seconde, le voile mou touche la paroi postérieure du pharynx, qu'il en soit repoussé par un courant d'air venu du nez, puis qu'une force quelconque le ramène contre le palais. Dans le ronflement délibéré, cette force est la contraction volontaire. Dans le ronflement nocturne passif en position couchée, cette force est celle de la pesanteur, et quand elle s'applique sur un voile volumineux et long, celui-ci tend spontanément à se coller sur le pharynx.

A cause des réactions de défense provoquées par cette gêne inspiratoire, se crée alors un cercle vicieux comportant une majoration de l'effort inspiratoire sous l'effet de cette obstruction, destinée à lutter contre l'asphyxie que celui-ci entraîne, mais responsable à son tour d'une obstruction qui est d'autant plus rapide et plus importante que cet effort est puissant. Ce contrôle volontaire étant impossible lors du sommeil, un ronflement à partir du moment où il s'installe ne peut que s'aggraver jusqu'à ce que l'éveil ou le semi éveil, redonnant du tonus aux muscles de l'oropharynx, supprime le rétrécissement et ses effets nocifs.

Outre le ronflement, l'effort respiratoire intense que le ronfleur doit effectuer provoque une irritation du voile contre la paroi du pharynx. C'est pourquoi le ronflement conduit à la formation d'un oedème de la luette qui s'installe de manière permanente (jour et nuit) au cours du temps.

L'apparition du ronflement est due à plusieurs facteurs indépendants les uns des autres, à savoir l'existence d'un voile trop long, un endormissement sur le dos et un rétrécissement du rhino-pharynx suite au relâchement des tissus et à l'oedème.

Le premier facteur responsable de l'apparition du ronflement est l'existence d'un voile trop long causée par une hypertrophie du voile : la luette s'allonge, les piliers postérieurs des amygdales s'épaississent et s'élargissent en deux demi rideaux prolongeant le voile vers le bas. Cette hypertrophie est liée à l'embonpoint qui infiltre de graisse les tissus, mais aussi à l'âge. Ce phénomène s'installe dès la quarantaine. Il reste en revanche exceptionnel avant cet âge, le ronflement étant dans ce cas lié à l'existence d'un voile naturellement très développé.

Le deuxième facteur responsable de l'apparition du ronflement est le décubitus dorsal (la position sur le dos du corps endormi). Cette position entraîne le voile détendu à reposer sur la paroi postérieure du pharynx. Dans cette position anatomique, le passage de l'air inspiratoire nasal peut facilement soulever le voile, le faire flotter et vibrer. A contrario, lorsque le dormeur est couché sur le ventre, le voile tombe en avant de sorte que le risque de toucher la paroi postérieure ou même latérale du pharynx est supprimé ; ainsi dégagé, le voile laisse grand ouvert derrière lui le couloir aérien qui lui est postérieur, l'air passant facilement.

Le dernier élément responsable du ronflement concerne le rétrécissement de la cavité buccale. Ce rétrécissement est entraîné par l'obésité, l'obstruction nasale et le rétrognathisme.

L'obésité est particulièrement fréquente chez les ronfleurs. Elle provoque rapidement, même lorsqu'elle est légère, un rétrécissement de l'espace libre du pharynx. En effet, le sujet en surcharge pondérale ne développe pas seulement son embonpoint vers l'extérieur, mais aussi vers les espaces intérieurs naturellement libres, à savoir essentiellement la bouche, le cou et les régions entourant les voies respiratoires hautes. Le pharynx congestif du sujet en surcharge pondérale de la cinquantaine est très caractéristique, énorme et comblé, rouge et gonflé. L'alcool pris au dîner favorise également le ronflement en congestionnant toutes les muqueuses aéro-digestives supérieures, c'est-à-dire notamment celles du nez et du pharynx, et en provoquant un relâchement des tissus par ses propriétés anti-spasmodiques. Par un mécanisme analogue, le ronflement est aggravé par les somnifères. D'autres facteurs que l'obésité ou l'alcool peuvent également concourir à ce rétrécissement de l'espace pharyngé. Il peut s'agir par exemple de certaines hypertrophies pathologiques de la langue.

Le rétrognathisme est une brièveté de la mandibule qui, de manière plus ou moins importante, rétrécit le pharynx dans le sens antéro-postérieur, et favorise ainsi la vibration de la luette et des parties molles. En effet, lorsque la mâchoire inférieure est courte et que le menton est projeté en arrière, la langue dont les points d'attache se trouvent ainsi repoussés en arrière, bascule et gêne plus facilement la respiration.

Le ronflement étant un phénomène physiologique particulièrement perturbant pour l'entourage du ronfleur et de grande ampleur dans la population, depuis ces dernières années un certain nombre de produits ont été développés. Cependant, les produits développés et à ce jour sur le marché ne sont pas totalement satisfaisants en terme de résultat sur le ronflement, mais également d'effets secondaires.

Tout d'abord, une majorité de ces produits sont délivrés par pulvérisation ou projection sur les muqueuses du pharynx et ont comme principal objectif la lubrification de ces muqueuses. La lubrification est obtenue alors par la présence d'huiles alimentaires dans ces compositions. Or, cette technique présente deux inconvénients majeurs.

Le premier inconvénient se rapporte au risque de passage au niveau des poumons de gouttelettes d'huiles responsables de pneumopathies huileuses qui représente un effet indésirable aux conséquences grave pour la santé.

Le second inconvénient concerne la durée d'action de ces produits. Le mélange est en effet pulvérisé sur la couche de mucus, qui présente une vélocité intrinsèque et entraîne le produit vers l'estomac, en dehors de son site d'action. Le phénomène naturel de déglutition et de production de salive renforce ce phénomène d'élimination des huiles de leur site d'action. C'est la raison pour laquelle ces produits ne sont satisfaisants que quelques minutes seulement en début de nuit, mais en aucun cas sur toute la durée de la nuit et plus particulièrement en milieu de nuit, lors du sommeil profond, et vers la fin de la nuit.

Ces observations sont confirmées par les résultats expérimentaux suivants.

Une spécialité sous forme de préparation liquide à base de liposomes ayant pour principale activité une action contre le ronflement a été testée. Cette préparation contient des huiles alimentaires (olive, tournesol...) encapsulées dans des liposomes à base de lécithine de soja. Cette préparation administrée lors du coucher a pour but de lubrifier l'arrière gorge pour faciliter le passage de l'air lors de la respiration en position horizontale. Un certain nombre d'essais in vitro ont été réalisés pour mettre en évidence la persistance ou non du produit au cours du temps sur une membrane biologique. La substance de référence choisie a été l'eau distillée, considérée comme non adhésive sur des supports physiques ou biologiques.

Trois types de tests ont été réalisés : la mesure d'étalement sur une membrane biologique, le test d'immersion dans la salive artificielle et le test de relargage. Le temps d'étalement est le temps que met un volume défini de liquide à parcourir 10 centimètres sur un plan incliné à 45°C. Le test d'immersion à la salive a pour but de mettre en évidence le temps de contact du produit avec la membrane biologique lorsqu'il est soumis à l'action de la salive. Enfin, le test de relargage a pour but de mettre en évidence le temps que met le produit à relarguer un traceur lorsqu'il est soumis à un « lessivage » permanent par la salive. Ces trois tests sont parfaitement décrits dans la littérature scientifique et sont des tests de référence lorsque l'on veut mesurer le maintien d'une solution sur son site d'action, dans des conditions expérimentales proches des conditions physiologiques de la muqueuse du pharynx.

Les résultats sont très clairs et évidents. Pour les trois tests, le produit à base de liposomes est comparable dans ses temps d'écoulement à l'eau distillée, c'est-à-dire que le produit ne reste pas en place sur son site d'action. La mesure du temps de maintien du produit sur son site d'action dans les trois tests est inférieure à la seconde. Ces résultats expérimentaux confirment les observations sur des ronfleurs. Le produit à base d'huiles alimentaires encapsulées dans des liposomes ne reste pas en place sur son site d'action, et ne peut ainsi prétendre à une quelconque efficacité concernant la lubrification des parois du pharynx.

Enfin, aucun de ces produits ne traite les composantes inflammatoires, oedème et relâchement des tissus qui sont observées chez les gros ronfleurs et qui sont inhérentes chez ces sujets.

La présente invention entend donc remédier aux inconvénients de l'art antérieur en proposant une combinaison d'actifs et de tout autre agent qui vont agir au niveau des muqueuses du pharynx de façon à diminuer, voire supprimer, le bruit du ronflement de façon certaine et mesurable durant toute la durée du sommeil.

Pour ce faire, la présente invention a pour objet de proposer une composition présentant des propriétés d'adhésion aux cellules mucocilliaires afin de limiter l'action d'élimination des actifs de la composition engendrée par la vélocité du mucus, et notamment de limiter l'élimination des actifs permettant la lubrification des parois de la muqueuse du pharynx.

La présente invention a également pour objet de proposer une composition permettant de traiter les effets secondaires provoquée par le ronflement, ces effets secondaires contribuant à leur tour à l'augmentation du ronflement. Il s'agit de l'inflammation et des oedèmes des tissus du pharynx ainsi que du relâchement de la muqueuse du pharynx.

La présente invention a également pour objet de proposer une composition pour lutter contre le ronflement, charactérisée en ce qu'elle comprend
- au moins une substance lubrifiante étant un agent tensioactif comprenant du glycérol, et
- au moins une substance bioadhésive étant un polysaccharide appartenant à la famille des carraghénanes.

La présente invention a également pour objet de proposer l'utilisation d'une composition selon les revendications pour la préparation d'une composition déstinée à lutter contre le ronflement.

Pour ce faire, la présente invention est remarquable, dans son acception la plus large, en ce qu'elle comprend au moins une substance lubrifiante destinée à lubrifier les parois des muqueuses du pharynx et au moins une substance bioadhésive apte à faire adhérer ladite substance lubrifiante aux cellules mucocilliaires situées au niveau du pharynx.

De manière surprenante, les inventeurs ont constaté que les composés entrant dans la composition selon l'invention, présentent des modes d'action complémentaires offrant ainsi une synergie pour une action plus efficace.

Particulièrement les inventeurs ont mis en évidence la synergie s'opérant entre la substance lubrifiante et la substance bioadhésive pour une bioadhésivité plus efficace.

Par l'expression « substance bioadhésive », on entend une substance biologique ou synthétique apte à « coller » à une muqueuse.

Pour bien comprendre la synergie s'opérant entre la substance lubrifiante et la substance bioadhésive aux fins d'empêcher le ronflement, ce dernier étant en rapport avec les caractéristiques anatomiques et mécaniques du pharynx et du mucus, il va être rappelé dans ce qui suit les caractéristiques physique et physiologique du mucus.

Le mucus constitue une couche visqueuse superficielle des tissus du pharynx.

La fonction du mucus est de capter les particules inhalées par la respiration (poussières, virus, bactéries, champignons...), de permettre leur élimination vers l'estomac, de protéger la muqueuse des voies respiratoires contre les irritants ou la dessiccation. De plus, le mucus joue un rôle très important dans les mécanismes d'immunité locale. Le mucus, qui constitue la première barrière à l'entrée dans l'organisme d'éléments étrangers est en renouvellement permanent afin d'éliminer le plus rapidement possible toutes les particules inhalées ou ingérées. Le renouvellement permanent du mucus est donc un facteur d'élimination rapide des ingrédients délivrés dans le pharynx.

Le mucus est composé de deux couches, à savoir :
- Une couche visqueuse superficielle, qui capte les particules, formée à 60-70% de mucines (substances insolubles diverses secrétées par cellules caliciformes et glandes sous-muqueuses) et à 30-40% de protéines (surtout des molécules impliquées dans l'immunité telles que transferrine, lysozyme, interféron, kallicréine, immunoglobulines...) ;
- Une couche fluide profonde composée d'éléments hydrosolubles et formant un milieu aqueux permettant le battement des cils, qui propulsent la couche superficielle vers le pharynx.

Une des propriétés du mucus est la clairance mucocilliaires, action combinée du mucus et des cils de la muqueuse respiratoire pour éliminer les particules étrangères. Cette action est la conséquence des cils. Ces cils baignent dans la couche fluide profonde, et comportent à leurs extrémités une petite structure ressemblant à un crochet, qui s'implante dans la couche visqueuse superficielle du mucus pendant le mouvement. Les cils battent de façon synchrone, le mouvement se faisant rapidement à l'aller (vers le pharynx), et plus lentement au retour. Les cils battent à environ 1.000 coups/min, et poussent le mucus vers le pharynx à une vélocité d'environ 5-20 mm/min.

Ainsi, les actifs d'un produit classique projeté sur le mucus parcourent en moyenne 1 cm par minute. Il faut donc en moyenne 15 minutes pour que la couche de mucus contenant les actifs ait quitté son site d'action au niveau du pharynx et perde ainsi son efficacité.

Par la présence d'une substance favorisant la bioadhésion, non pas sur le mucus, mais sur les cellules mucocilliaires, de la substance lubrifiante, laquelle favorise le glissement du voile sur les parois du pharynx, l'efficacité de la composition contre le ronflement peut être obtenue, à la différence des produits de l'art antérieur, sur une longue période.

Afin de favoriser l'adhésion des actifs, et notamment de l'actif lubrifiant, la substance bioadhésive est choisie dans le groupe comprenant : des polysaccharides, des dérivés de la cellulose, des dérivés acryliques ou des dérivés de protéines, ou de tout autre agent connu à ce jour comme agent bioadhésif.

Afin de renforcer la propriété de bio-adhésion, la substance bioadhésive est avantageusement un polysaccharide (polymère naturel) appartenant à la famille des carraghénanes.

Les carraghénanes sont des polysaccharides qui constituent les parois cellulaires de divers algues rouges (Rhodophycées) appartenant aux familles des Gigartinaceae, Hypneaceae, Furcellariaceae et Polyideaceae. Ils comportent des longues chaînes galactanes, polyélectrolytes anioniques. Leur masse moléculaire peut être supérieure à 10⁶ Daltons.

Ces polymères linéaires, formés par des motifs disaccharides [AB], sont composés par deux unités D-galactoses liées alternativement par des liaisons (1 → 3) et (1 → 4). Ce sont des polysaccharides très sulfatés (20-50%) et les résidus galactoses peuvent être sous forme 3,6-anhydro.

La fonction sulfate des carraghénanes est une fonction particulièrement réactive qui donne naissance à des complexes. Les carraghénanes réagissent entre autre avec des protéines, conférant auxdites protéines des propriétés bio-adhésives. Ainsi, les carraghénanes se combinent avec les protéines contenues dans les muqueuses du pharynx.

Plus précisément, la propriété de bioadhésion est générée par une interaction entre les polysaccharides et le mucus recouvrant les muqueuses du pharynx. En effet, de part, leur nature chimique (polymères de haut poids moléculaires), les polysaccharides sont avides d'eau. Au contact du mucus, lequel est hautement hydraté et présente une certaine viscosité due à la présence de la mucine, les polysaccharides gonflent rapidement avec création de liaisons hydrogènes entre les groupements hydrophiles du polymère et ceux de la mucine. Cette propriété de bioadhésion est alors renforcée par la formation de liaisons supplémentaires du fait de la fonction sulfate des carraghénanes. En effet, les groupements sulfates des carraghénanes, particulièrement réactifs, donnent naissance à des réactions de complexation entre les groupements sulfates du polysaccharide et les atomes d'azote de la mucine, formant ainsi des liaisons supplémentaires aux liaisons hydrogène, dites liaisons de complexation.

Préférentiellement, on utilise dans l'invention des lambda- et iota-carraghénanes.

L'avantage des carraghénanes est donc de permettre aux actifs contenus dans la composition anti-ronflement, et notamment à la substance lubrifiante, de rester sur leur site d'action pendant un long moment (durée supérieure ou égale à 8 heures).

Concernant la lubrification des parois des muqueuses du pharynx, celle-ci est idéalement assurée au moyen d'un agent tensioactif, ou de tout autre agent ou combinaison d'agents lubrifiants connue à ce jour.

Les tensioactifs ou agents de surface sont des molécules d'origine naturelle ou synthétique possédant d'une part une chaîne à caractère lipophile (ou partie hydrophobe) et d'autre part un groupement à caractère hydrophile (partie polaire). Ces composés sont dits amphiphiles.

Avantageusement, la partie polaire de l'agent tensioactif est choisie dans le groupe comprenant du glucose, du sucrose, du lactose, du glycérol, du xylose, des peptides, des acides aminés, des nucléotides, la partie hydrophobe étant choisie dans le groupe comprenant des acides gras, un alcool gras, une amine grasse, des esters, des glycérides, des phospholipides.

Dans un mode de réalisation préférée de l'invention, la partie hydrophobe est constituée de phosphatidylcholine qui fait partie de la famille des phospholipides qui sont des composés majeurs des membranes entourant les cellules. Comme elle contribue au maintien de l'intégrité de ces membranes et à leur « réparation », elle joue un rôle essentiel dans le bon fonctionnement de l'organisme. Par ailleurs, la phosphatidylcholine est un composant majeur de la bile et joue un rôle dans le métabolisme des gras.

Selon un mode de réalisation avantageux de l'invention, la composition comprend en outre un ou plusieurs agent(s) anti-inflammatoire(s) et/ou tonique(s) des muqueuses et des tissus.

L'incorporation de ces actifs dans la substance bioadhésive sera avantageusement favorisée par les propriétés émulsifiantes de la substance lubrifiante choisie, et notamment de la phosphatidylcholine.

Afin de diminuer l'inflammation et l'oedème des tissus du pharynx, l'agent anti-inflammatoire choisi est avantageusement un extrait de *Filipendula ulmaria,* et de préférence un extrait de sommités fleuris de *Filipendula ulmaria* lesquelles renferment un ensemble de composants dont des flavonoïdes et autres hétérosides phénoliques.

Afin d'augmenter la tonicité de la muqueuse et des tissus du pharynx, sont utilisés préférentiellement des extraits de plantes riches en tannins de sorte à conférer des propriétés d'astringence sur les muqueuses et les tissus du pharynx. Parmi ces plantes, l'agent tonique choisi est avantageusement du cynorrhodon. Le cynorrhodon est un fruit allongé d'un arbrisseau dénommé Rosa canina (églantier).

Il est bien entendu évident que les extraits choisis en tant qu'agents anti-inflammatoires et toniques, et mentionnés ci-dessus, sont donnés à titre d'exemple. Il pourra en effet être employé dans la composition objet de la présente invention tout autre agent permettant de diminuer l'inflammation et l'oedème et/ou d'augmenter la tonicité de la muqueuse et des tissus du pharynx.

Ainsi, la composition selon l'invention comprend des actifs choisis ayant des propriétés de lubrification des muqueuses du pharynx sur une longue période, associées à un renforcement de la tonicité des muqueuses et à une action anti-inflammatoire.

Il est bien entendu évident que les substances choisies seront des substances à caractère hypoallergénique.

Par ailleurs, ladite composition comprendra avantageusement un excipient ou présentera une formulation générale, adapté au mode d'administration envisagé. À titre préférentiel, la composition selon l'invention se présente sous la forme d'une mousse délivrée par voie buccale. Une mousse peut s'obtenir par dispersion d'un gaz dans un faible volume de liquide provoquant l'apparition de bulles dont la taille peut varier entre 50 µm et plusieurs millimètres.

La mousse constitue en effet, au regard des compositions classiquement administrées sous forme liquide, une forme galénique spécifique adaptée pour une imprégnation optimale des muqueuses.

En effet, la mousse possède des propriétés d'étalement sur les muqueuses supérieures à la forme liquide qui a tendance, dès sa projection dans la bouche, à couler au fond de la gorge.

Du fait même de sa structure, la mousse permet d'augmenter considérablement la surface de contact entre deux surfaces incompatibles. En effet, une des difficultés rencontrées dans la conception d'une formulation liquide à disperser sur une membrane biologique est souvent l'incompatibilité entre les deux surfaces. Dans le cas précis des tissus du pharynx, ceux-ci sont recouverts d'une couche de mucine présentant des propriétés hydrophobes. La mucine est une protéine qui assure une fonction structurale de protection dans les voies aériennes et digestives principalement. Or, les carraghénanes pour interagir avec la mucine et les cellules épithéliales doivent être placés en solution aqueuse, incompatible physiquement avec la couche de mucine. La forme mousse présente ainsi l'avantage de permettre un contact entre les carraghénanes de la solution et les cellules épithéliales, permettant ainsi que renforcer la bioadhésion. Des tests montre ainsi dans plusieurs expériences que la forme mousse présente un pouvoir bioadhésif supérieur à la forme liquide délivrée en mêmes quantités. Par ailleurs, des tests ont montré que la forme mousse "tapisse" mieux les muqueuses de la gorge et du pharynx. Ces mêmes tests montrent également que le temps de contact entre la mousse et les muqueuses est supérieur à celui d'une forme liquide.

Un autre avantage est un maintien plus long dans le temps de la structure de la mousse grâce par exemple à certaines molécules contenues dans les extraits de plantes associés à la formulation du produit. Certains types de molécules contenues dans les extraits de plantes utilisés dans la formulation (extraits de *Filipendula Ulmaria* et de *Rosa canina*)favoriseraient la stabilité dans le temps de la forme mousse par des phénomènes d'interactions physico-chimiques. Ainsi, une forme mousse serait plus stable dans le temps grâce à l'action de composés de type flavonoïdes. Cette stabilité dans le temps vient renforcer la propriété énumérée ci-dessus en permettant un temps de contact plus long entre la solution et les cellules épithéliales des muqueuses du pharynx.

Enfin, la forme mousse présente un autre avantage qui est de ne pas diffuser de gouttelettes dans les voies respiratoires, comme c'est le cas dans les formes classiques de spray pulvérisant des liquides. Ces gouttelettes sont en effet responsables de dommages au niveau des poumons et des bronches. Ainsi, avec des préparations huileuses dispersées sous forme de spray, on a constaté l'apparition de pneumopathies huileuses aux conséquences très dommageables pour la santé des individus. Les pneumopathies huileuses sont des pneumopathies diffuses, pausé, symptomatiques, avec aspects radio-nodulaires, prédominant aux bases ou des pneumopathies localisées pseudo-tumorales ou granulome huileux. Le pronostic est grave et l'évolution irréversible. Par une pulvérisation de mousse, on évite ainsi tout risque de passage de solution au niveau des voies respiratoires.

Par ailleurs, la mousse diffusée est considérée par bon nombre de patients comme plus acceptable et plus facile à utiliser par rapport à une forme liquide. Or dans le domaine du ronflement où un traitement quotidien est nécessaire, l'acceptabilité pour les patients de ce type de traitement est un facteur très important de l'observance et donc du succès clinique.

La mousse constitue donc une forme galénique préférée de la composition anti-ronflement selon l'invention. Cependant, il est bien entendu évident que la composition anti-ronflement ne se limite en aucune manière à cette forme galénique spécifique.

La composition pourra notamment se présenter sous forme solide telle que sous forme de comprimés, pastilles, pâte à mâcher ou à délitement ou tout autre forme solide à délitement instantané.

Bien qu'il s'agisse d'une forme moins avantageuse comme précisé plus haut, la composition pourra également se présenter sous forme liquide comme par exemple sous forme de gel, de pâte, de dentifrices, de solutions pour la bouche, etc.

Le pourcentage en poids de chacun des actifs présents dans la composition anti-ronflement objet de l'invention est le suivant :
- substance bioadhésive : de 0,5 à 20% environ,
- substance lubrifiante : de 0,5 à 20% environ,
- agent anti-inflammatoire : de 0,5 à 8% environ,
- agent tonique : de 0,5 à 8% environ.

Il est à noter que, selon la forme dans laquelle la composition est délivrée, le pourcentage en poids de la substance bioadhésive différera. En effet, dans le cas où la composition se présente sous forme de mousse, elle comprendra environ de 1 à 5% de substance bioadhésive. Dans le cas où la composition se présente sous la forme solide, elle comprendra environ de 5 à 20% de substance bioadhésive.

Ci-dessous est donné à titre d'exemple la formulation d'une composition selon l'invention se présentant sous forme de mousse.

| | |
|---|---|
| Carraghénanes | de 1 à 5% environ |
| | *de préférence 3%* |
| Phosphatidylcholine | de 1 à 10% environ |
| | *de préférence 5%* |
| Reine des près (extraits) | de 1 à 5% environ |
| | de préférence 3% |
| Cynorrhodon | de 1 à 5% environ |
| | *de préférence de 2%* |
| Excipients : édulcorants, arômes, conservateurs, eau | |

La composition selon l'invention (Préparation Bioadhésive) a été étudiée expérimentalement afin de vérifier ses propriétés de bioadhésion et de les comparer à une substance de référence non bioadhésive (eau distillée) et une préparation commercialisée (Préparation Liposomes) à base de liposomes. Les mesures qui ont été réalisées sont le temps d'écoulement sur une membrane biologique et le test d'immersion à la salive artificielle.

Le temps d'étalement est le temps que met un volume défini de liquide à parcourir 10 cm sur un plan incliné à 45°C. Ce test a été réalisé à 3 niveaux :
- sur une plaque inox de 20 cm², membrane non biologique.
- sur une plaque inox de 20 cm² recouverte d'une membrane de cellulose, membrane biologique.
- sur une plaque inox de 20 cm² recouverte d'une membrane de cellulose elle-même imprégnée par une solution à 5% de mucine.

Pour cette étude, un dépôt de 0,5 ml a été réalisé à 0,5 cm du bord supérieur de la plaque, le dépôt étant fait en position horizontale. A 5 cm du bord supérieur de la plaque est matérialisée la ligne de déclenchement du chronomètre lorsque le liquide la franchit après avoir incliné la plaque à 45°C. De même, à 5 cm du bord inférieur de la plaque est matérialisé la ligne d'arrêt du chronomètre lorsque le liquide franchit cette dernière. Les temps mesurés sont les suivants :

| | Support inox | Inox + membrane | Inox + membrane + mucine |
|---|---|---|---|
| Eau distillée | < 1" | < 1" | < 1" |
| Préparation Liposomes | < 1" | < 1" | < 1" |
| **Préparation Bioadhésive** | **1'20 +/- 3"** | **3,27 +/- 10"** | **7,08 +/- 27"** |

Ce tableau montre que la Préparation Liposome se comporte comme l'eau distillée, que ce soit sur de l'inox ou sur membrane biologique pure ou additionnée de mucine. Au contraire, la Préparation Bioadhésive montre clairement des propriétés de bioadhésion, notamment sur le test utilisant de la mucine qui simule la muqueuse buccale et du pharynx.

Le test d'immersion dans la salive artificielle a pour but de mettre en évidence le temps de contact du produit avec la membrane biologique lorsqu'il est soumis à l'action de la salive. Comme précédemment, le produit est déposé à la surface de la membrane biologique recouverte de mucine. Après étalement sur la distance de 10 cm, l'ensemble plaque/membrane biologique/produit, est plongé à la verticale dans un récipient contenant 2 litres de salive artificielle. Le temps que le produit persiste à la surface de la membrane est déterminé à l'aide d'un chronomètre. Les temps mesurés sont les suivants :

| | |
|---|---|
| Eau distillée | < 1" |
| Préparation Liposomes | < 1" |
| **Préparation Bioadhésive** | **64'** |

En raison de la non adhésion de la Préparation Liposomes sur la membrane biologique recouverte de mucine, le temps d'immersion du produit dans la salive artificielle s'est révélé négatif (< 1"). Il y a une absence totale d'adhésion en présence de salive. Au contraire, pour la Préparation Bioadhésive, celle-ci montre d'excellentes propriétés de bioadhésion.

En modifiant le mode d'étalement du produit sur la plaque, il n'a pas été possible de déterminer un temps d'immersion de la Préparation Liposomes dans la salive, étant donné que le produit est immédiatement dispersé dans la salive artificielle. Au contraire, en plaçant la plaque en mode incliné à 45°, la Préparation Bioadhésive montre des propriétés d'adhésion supérieures à 48 heures.

La composition selon l'invention a été étudiée expérimentalement afin de vérifier la synergie s'opérant entre la substance lubrifiante et la substance bioadhésive sur ses propriétés de bioadhésion. L'étude réalisée est la mesure du temps d'étalement défini précédemment, en utilisant différentes concentrations d'un mélange de glycerol et d'eau à 3% (S2) comme substance lubrifiante pour une concentration donnée de carraghénanes (S1) comme substance bioadhésive.

Ce test a été réalisé sur une plaque inox de 20 cm² recouverte d'une membrane de cellulose elle-même imprégnée par une solution à 5% de mucine.

Pour cette étude, un dépôt de 0,5 ml a été réalisé à 0,5 cm du bord supérieur de la plaque, le dépôt étant fait en position horizontale. À 5 cm du bord supérieur de la plaque est matérialisée la ligne de déclenchement du chronomètre lorsque le liquide la franchit après avoir incliné la plaque à 45°C. De même, à 5 cm du bord inférieur de la plaque est matérialisée la ligne d'arrêt du chronomètre lorsque le liquide franchit cette dernière. Les résultats sont les suivants :

| | Temps d'écoulement (en min) | Rapport temps d'écoulement / concentration en S2 |
|---|---|---|
| Solution de carraghénanes à 2% (S1) | 0.83 | / |
| S1 + S2 (mélange de glycerol et d'eau à 3%) à 7.5% | 14 | 1.9 |
| S1 + S2 à 10% | 18.6 | 1.9 |
| S1 + S2 à 16% | 70 | 4.4 |

Le rapport entre le temps d'écoulement et la concentration en S2 montre une constance (1.9) pour des concentrations de S2 jusqu'à 10%, puis une augmentation sensible pour des concentrations de l'ordre de 16%. Ceci montre clairement que pour une solution donnée contenant 2% de carraghénanes, l'addition de glycerol augmente de manière exponentielle le temps d'écoulement, qui est une mesure de la bioadhésivité du mélange final.

Ce tableau montre clairement l'effet potentialisateur du glycerol sur la bioadhésivité des carraghénanes.

L'invention est décrite dans ce qui précède à titre d'exemple. Il est entendu que l'homme du métier est à même de réaliser différentes variantes de l'invention sans pour autant sortir du cadre du brevet.

## Revendications

1. Composition pour lutter contre le ronflement, **caractérisée en ce qu'**elle comprend :
- au moins une substance lubrifiante, ladite substance lubrifiante étant un agent tensioactif comprenant du glycérol, et
- au moins une substance bioadhésive, ladite substance bioadhésive étant un polysaccharide appartenant à la famille des carraghénanes.

2. Composition selon la revendication 1, **caractérisée en ce qu'**elle contient de 0,5 à 20% de carraghénanes.

3. Composition selon la revendication 2, **caractérisée en ce qu'**elle contient de 1 à 5% de carraghénanes.

4. Composition selon l'une quelconque des revendications 1 à 3, **caractérisée en ce que** l'agent tensioactif comprend également de la phosphatidylcholine.

5. Composition selon l'une quelconque des revendications précédentes, **caractérisée en ce qu'**elle contient de 0,5 à 20% de substance lubrifiante.

6. Composition selon l'une quelconque des revendications précédentes, **caractérisée en ce qu'**elle comprend en outre un ou plusieurs agent(s) anti-inflammatoire(s) et/ou tonique(s) des muqueuses et des tissus du pharynx.

7. Composition selon la revendication 6, **caractérisée en ce que** l'agent anti-inflammatoire est un extrait de Filipendula ulmaria.

8. composition selon la revendication 7, **caractérisée en ce que** ledit extrait est un extrait de sommités fleuries de Filipendula ulmaria.

9. Composition selon l'une quelconque des revendications 6 à 8, **caractérisée en ce qu'**elle contient de 0,5 à 8% environ d'agent anti-inflammatoire.

10. Composition selon la revendication 6, **caractérisée en ce que** l'agent tonique des muqueuses et des tissus du pharynx est du cynorrhodon.

11. Composition selon la revendication 6 ou la revendication 10, **caractérisée en ce qu'**elle contient entre 0,5 et 8% d'agent tonique.

12. Composition selon l'une quelconque des revendications précédentes, **caractérisée en ce qu'**elle se présente sous la forme d'une mousse.

13. Utilisation d'une composition selon l'une quelconque des revendications 1 à 12 pour la préparation d'une composition destinée à lutter contre le ronflement.

## Claims

1. An anti-snoring composition **characterized in that** it includes:
- at least a lubricant substance, said lubricant substance being a surfactant comprising glycerol, and
- at least a bioadhesive substance, said bioadhesive substance being a polysaccharide belonging to the carrageenan family.

2. The composition according to claim 1, **characterized in that** it contains from 0.5 to 20% of carrageenans.

3. The composition according to claim 2, **characterized in that** it contains from 1 to 5% of carrageenans.

4. The composition according to any one of claims 1 to 3, **characterized in that** the surfactant also comprises phosphatidylcholine.

5. The composition according to any one of the preceding claims, **characterized in that** it contains from 0.5 to 20% of a lubricant substance.

6. The composition according to any one of the preceding claims, **characterized in that** it further comprises one or several anti-inflammatory and/or tonic agent(s) of the mucusa and tissues of the pharynx.

7. The composition according to claim 6, **characterized in that** it the anti-inflammatory agent is an extract of Filipendula ulmaria.

8. The composition according to the claim 7, **characterized in that** said extract is an extract of flowering tops of Filipendula ulmaria.

9. The composition according to any one of claims 6 to 8, **characterized in that** it contains from about 0.5 to 8% of an anti-inflammatory agent.

10. The composition according to claim 6, **characterized in that** the tonic agent of the mucosa and the tissues of the pharynx is rose hip.

11. The composition according to claim 6 or claim 10, **characterized in that** it contains between 0.5 and 8% of a tonic agent.

12. The composition according to any one of the preceding claims, **characterized in that** it is under the form of a foam.

13. Use of a composition according to any one of claims 1 to 12 for the preparation of an anti-snoring composition.

## Patentansprüche

1. Verbindung zur Bekämpfung des Schnarchens, **dadurch gekennzeichnet, dass** sie Folgendes umfasst:
- wenigstens eine Gleitsubstanz, wobei die genannte Gleitsubstanz ein Glycerin umfassendes oberflächenaktives Mittel ist, und
- wenigstens eine bioadhäsive Substanz, wobei die genannte bioadhäsive Substanz ein zur Familie der Carrageene gehörendes Polysaccharid ist.

2. Verbindung gemäß Anspruch 1, **dadurch gekennzeichnet, dass** sie 0,5 bis 20 % Carrageene enthält.

3. Verbindung gemäß Anspruch 2, **dadurch gekennzeichnet, dass** sie 1 bis 5 % Carrageene enthält.

4. Verbindung gemäß Anspruch 1 bis 3, **dadurch gekennzeichnet, dass** das oberflächenwirksame Mittel ebenfalls Phosphatidylcholin enthält.

5. Verbindung gemäß einem der vorherigen Ansprüche, **dadurch gekennzeichnet, dass** sie 0,5 bis 20 % Gleitmittel enthält.

6. Verbindung gemäß einem der vorherigen Ansprüche, **dadurch gekennzeichnet, dass** sie darüber hinaus ein oder mehrere entzündungshemmende(s) und / oder stimulierende(s) Mittel der Schleimhäute und der Gewebe der Rachenhöhle umfasst.

7. Verbindung gemäß Anspruch 6, **dadurch gekennzeichnet, dass** das entzündungshemmende Mittel ein Extrakt aus Filipendula Ulmaria ist.

8. Verbindung gemäß Anspruch 7, **dadurch gekennzeichnet, dass** der genannte Extrakt ein Extrakt aus in Blüte stehenden Spitzen von Filipendula Ulmaria ist.

9. Verbindung gemäß Anspruch 6 bis 8, **dadurch gekennzeichnet, dass** sie rund 0,5 bis 8 % entzündungshemmendes Mittel enthält.

10. Verbindung gemäß Anspruch 6, **dadurch gekennzeichnet, dass** das die Schleimhäute und die Gewebe der Rachenhöhle stimulierende Mittel Hagebutte ist.

11. Verbindung gemäß Anspruch 6 oder Anspruch 10, **dadurch gekennzeichnet, dass** sie zwischen 0,5 und 8 % stimulierendes Mittel enthält.

12. Verbindung gemäß einem der vorherigen Ansprüche, **dadurch gekennzeichnet, dass** sie die Form eines Schaums aufweist.

13. Verwendung einer Verbindung gemäß Anspruch 1 bis 12 für die Zubereitung einer Verbindung, die zur Bekämpfung des Schnarchens bestimmt ist.
